# FASCICULE DE BREVET EUROPEEN

(11) **EP 1 971 859 B1**
(45) Date de publication et mention de la délivrance du brevet: **14.02.2018**
(21) Numéro de dépôt: 06847153.1
(22) Date de dépôt: 22.12.2006
(51) Int. Cl.: G01N 33/08, B41J 3/407, A01K 43/10, B07C 5/342, B07C 5/38

(54) **PROCEDE ET INSTALLATION DE MIRAGE DES OEUFS**
VERFAHREN UND EINRICHTUNG ZUM DURCHLEUCHTEN VON EIERN
METHOD AND SYSTEM FOR CANDLING EGGS

(30) Priorité: 23.12.2005 FR 0513252; 03.01.2006 FR 0600018
(43) Date de publication de la demande: 24.09.2008
(73) Titulaire: Visio Nerf, 49340 Nuaille (FR)
(72) Inventeur: ROBERT, Pierre, 49300 Cholet (FR); SOMVILLE, Olivier, 49300 Cholet (FR)
(74) Mandataire: Thibon-Littaye, Annick
(86) Numéro de dépôt international: PCT/FR2006/002895
(87) Numéro de publication internationale: WO 2007/071851

(56) Documents cités:
- EP-A- 1 457 108
- WO-A-00/36411
- WO-A-01/56789
- WO-A-99/14589
- FR-A- 1 285 525
- JP-A- 9 127 096
- US-A- 3 540 824
- US-A- 4 805 778
- US-A- 4 843 958
- US-A- 5 017 003
- US-A- 5 898 488
- US-A- 5 900 929

## Description

La présente invention concerne les techniques d'examen par visiométrie quand elles portent sur des objets se présentant à l'examen en des localisations discrètes définies, se disposant par rangées successives, dans un défilement continu, suivant une répartition spatiale régulière périodiquement répétitive. La notion d'examen par visiométrie vise ici toutes les techniques de type opto-électronique qui impliquent une saisie d'image détectant un faisceau lumineux à partir de chaque objet soumis à exposition sous un rayonnement incident, ainsi qu'une analyse par traitement d'image effectuée sur les signaux obtenus, sous la commande de logiciels adaptés, pour en déduire l'état des objets examinés au regard de propriétés déterminées. Par ailleurs la notion d'objet s'étend, dans le cadre de l'invention, à ce qui apparaîtra plus loin comme une absence d'objet. Autrement dit, les objets considérés sont plutôt qualifiés d'emplacements, étant entendu que ces emplacements répondent à la disposition relative périodiquement répétitive en rangées successives déjà mentionnée.

Dans tout ce qui suit, on décrira différentes formes de mise en oeuvre de l'invention en faisant plus spécifiquement référence à ses applications préférées, qui se situent dans le domaine de l'industrie alimentaire, pour le mirage des oeufs de volailles. Les emplacements soumis à examen sous rayonnement sont alors, en pratique, les différentes alvéoles de casiers dans lesquels les oeufs sont rangés, chacun étant reçu dans l'une des alvéoles. D'autre part, les opérations de mirage des oeufs, telles qu'elles sont couramment mise en oeuvre à un stade intermédiaire de la production de poussins pour les oeufs de poule, entre un incubateur et un éclosoir, ont pour objet d'examiner les oeufs par transparence en les exposant individuellement à un faisceau lumineux, le plus souvent en lumière infrarouge, afin d'établir une distinction entre les oeufs en fonction d'un état de fécondation de chacun, et de permettre ainsi de sélectionner ceux qui sont fécondés, en excluant ceux qui ne le sont pas, et qu'il est donc inutile d'envoyer vers les éclosoirs où naîtront des poussins. Plus précisément, on attribue à chaque oeuf une qualité d'état fécondé ou d'état non fécondé en fonction de l'atténuation que subit un faisceau lumineux auquel il est exposé. Mais que ce soit pour des oeufs, pour tout autres produits en unités individualisées, ou même pour des emplacements discrets constitués par des zones adjacentes d'un produit continu, il sera manifestement à la portée de l'Homme de métier de transposer le vocabulaire pour appliquer l'invention à d'autres critères de discrimination et de sélection, ainsi qu'à d'autres domaines industriels.

Dans les installations de mirage des oeufs classiques, dont celles qui sont décrites notamment dans la demande internationale de brevet WO 99/14589 (Ecmas) ou dans le brevet américain US 5 900 929 (Embrex), on fait défiler en série des casiers qui contiennent chacun un lot d'oeufs. Il s'agit en général directement des casiers utilisés pour l'incubation des oeufs. Les oeufs y sont placés dans des alvéoles situées en des emplacements régulièrement répartis, dans chaque casier, suivant une répartition répétitive de lignes longitudinales et de rangées transversales. Les casiers sont placés l'un après l'autre horizontalement sur un dispositif de convoyage (par exemple du type à tapis circulant en boucle fermée) qui les entraîne à travers le poste d'examen opto-électronique.

Dans celui-ci, une source de rayonnement émet un flux lumineux incident qui éclaire individuellement chacun des oeufs. Quand il s'agit d'une application visant à situer la chambre d'air à l'intérieur des oeufs, ces moyens d'illumination éclairant les oeufs sont disposés du même côté que les détecteurs qui reçoivent la lumière émergeant des oeufs et déterminent sa composition d'après la modification induite par chacun d'eux. Il en serait de même si, par exemple, l'application consistait à examiner la coloration de fruits qui seraient individuellement maintenus, à la place des oeufs, dans les alvéoles de casiers similaires. Mais dans les applications plus courantes de mirage des oeufs visant à distinguer les oeufs fécondés des oeufs à éliminer de la chaîne en cours, comme il est pris en exemple ici, la source est en général disposée sous le circuit de convoyage des casiers, pour une illumination de bas en haut vers des détecteurs situés au-dessus. De façon avantageuse, bien que non impérative, on utilise une lumière dans la gamme des longueurs d'onde de l'infrarouge.

Pour les moyens détecteurs sensibles au flux émergent on peut utiliser soit des capteurs individuels respectivement associés à chaque oeuf, soit préférentiellement, une caméra vidéo. Lorsque l'intensité lumineuse détectée s'abaisse en dessous d'un seuil prédéterminé, témoignant d'une atténuation seuil qui peut être calculée de manière connue en fonction des propriétés de diffusion des oeufs ou déterminée expérimentalement, cela indique la présence d'un germe d'embryon, et le système est commandé pour attribuer automatiquement la qualité d'état fécondé à l'oeuf examiné. La structure alvéolaire des casiers d'incubation est naturellement adaptée à l'examen optique. En général les alvéoles sont sans fond, pour un examen par transmission, et elles maintiennent les oeufs avec leur grand axe vertical, ce qui se prête bien à un examen que l'on a intérêt à réaliser au voisinage de ce grand axe.

Les configurations des emplacements en lignes et rangées les plus courantes, notamment pour les alvéoles recevant des oeufs, sont soit à maille carrée, soit à maille triangulaire, ou plus particulièrement à maille hexagonale, dans ce qu'il est usuel d'appeler une disposition en quinconce. Les dispositions en quinconce diffèrent des dispositions à maille carrée par le fait que d'une rangée à l'autre, les alvéoles ne sont pas en ligne dans la direction longitudinale de déplacement, mais elles sont décalées latéralement. Par exemple, si la distance de décalage peut être une fraction quelconque du pas de répartition des alvéoles au sein de chaque rangée, la répartition en quinconce la plus fréquente correspond à un décalage d'un demi-pas, dans une répartition régulière de type à maille hexagonale.

Par ailleurs les moyens d'illumination et les moyens de détection associés sont disposés et pilotés en adéquation à la configuration spatiale des alvéoles des casiers. En exploitation industrielle, un compromis optimal entre cadence de traitement, fiabilité des tris, coûts d'installation et de mise en oeuvre, implique d'opérer simultanément sur un groupe d'emplacements d'oeufs de manière répétitive au cours du défilement à travers un poste d'examen visiométrique dans lequel le matériel reste fixe. C'est dire que d'une manière générale, l'examen a lieu rangée par rangée, au fur et à mesure que les rangées successives défilent sous les détecteurs. De ce point de vue, l'invention prévoit, comme on l'expliquera mieux plus loin, d'examiner les rangées décalées des dispositions en quinconce en les considérant groupées, pour utiliser les mêmes moyens d'illumination et moyens détecteurs, par exemple, sur la rangée d'ordre pair et la rangée d'ordre impair dans chaque couple de rangées d'une disposition à maille hexagonale.

De façon préférentielle, l'analyse par examen visiométrique visant à la détection de la présence d'oeufs fécondés a lieu en entrée de l'installation de mirage des oeufs. Les casiers sont posés manuellement ou automatiquement sur le dispositif de convoyage, par exemple un convoyeur à tapis circulant en boucle fermée, qui les entraîne à travers le poste d'examen visiométrique. En sortie de ce dernier, l'installation est avantageusement complétée par un poste de marquage, où les oeufs sont marqués sélectivement, pour qu'ils soient transférés ensuite vers des destinations réservées distinctes, suivant qu'ils sont fécondés ou non. Le tri est en général effectué manuellement pour éliminer de la chaîne les oeufs non fécondés, mais il peut aussi être effectué automatiquement par un appareillage supplémentaire.

L'invention vise à améliorer les conditions d'exploitation industrielle de telles installations de mirage des oeufs, principalement en ce qui concerne la fiabilité de la détection de l'état de fécondation des oeufs et la cadence de traitement. Spécialement dans le cas d'une installation dans laquelle les casiers d'oeufs sont traités en ligne dans un poste de marquage après le poste d'examen visiométrique, il se pose le problème de pouvoir marquer les oeufs avec efficacité et rapidité, en utilisant directement le signal produit par traitement numérique des images saisies par l'examen visiométrique, tout en opérant soigneusement pour ne pas risquer de briser les coquilles des oeufs. Dans le même souci d'un traitement à haute cadence en toute sécurité dans la sélection des oeufs en fonction de leur état de fécondation, il est utile de trouver solution à des difficultés qui apparaissent au niveau du poste d'examen visiométrique lorsqu'il se présente plus de deux états à distinguer pour chaque emplacement de l'ensemble en défilement, surtout si dans certaines rangées d'oeufs, des alvéoles sont restées vides par accident.

Suivant l'un de ses aspects, la présente invention prend en considération le fait que les mesures d'atténuation des faisceaux lumineux peuvent se trouver fortement perturbées par des phénomènes d'éblouissement des capteurs de la caméra lorsque les flux lumineux à détecter à un même moment sont de niveaux d'intensité trop différents et conduire de ce fait à des informations fausses concernant l'état des oeufs examinés. Cette situation se rencontre fréquemment, par exemple en présence d'un oeuf pourri ou d'une alvéole vide parmi des oeufs clairs et fécondés. On conçoit aisément que l'absence d'oeuf dans une alvéole, laissant passer tout le flux lumineux émis, se traduit par une illumination de très grande intensité, beaucoup plus élevée que quand un oeuf y est présent, quelque soit l'état de ce dernier. Accessoirement, quand c'est un oeuf clair qui est présent dans une alvéole déterminée, il atténue relativement peu le flux lumineux qui le traverse, mais l'atténuation de ce flux est sensiblement plus forte pour un oeuf à faux germe, un oeuf fécondé, un oeuf pourri, les trois cas étant ici cités dans l'ordre des atténuations croissantes.

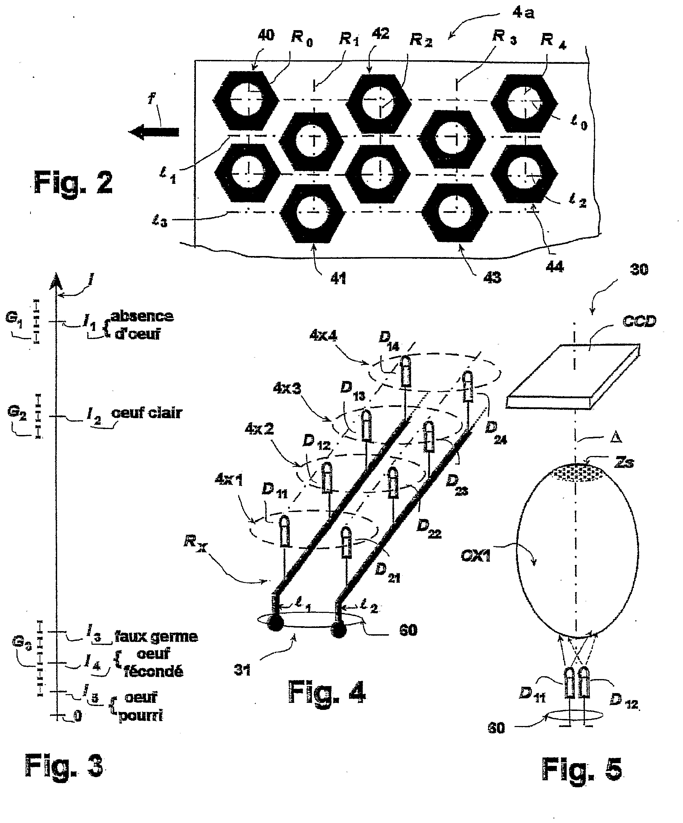

A titre d'exemple non limitatif, le diagramme de la figure 3, placé en fin de la présente description, illustre schématiquement l'échelle des intensités lumineuses / captées par la caméra de détection. Elles s'étalent sur trois gammes d'intensité lumineuses très distinctes :
- G₁ : illumination très élevée correspondant au cas "absence d'oeuf" (intensité *I*₁) ;
- *G₂* : illumination élevée correspondant au cas "oeuf clair" (intensité *I*₂) ;
- *G*₃ : illuminations faibles correspondant aux cas "faux germe" (intensité *I*₃), "oeuf fécondé" (intensité *I*₄) et "oeuf pourri" (intensité *I*₅).

Du fait de la présence de cette échelle d'intensités lumineuses très "dilatée", il existe un risque "d'éblouissement" du capteur de la caméra. En effet, dans les dispositifs modernes, on utilise le plus souvent un capteur monolithique à semi-conducteurs, du type dit "CCD" selon l'abréviation anglo-saxonne usuelle (pour "Charge Coupled Device" ou "dispositif à couplage de charges"). Un tel capteur peut être constitué d'une barrette d'éléments photo-détecteurs ou photosites. Ces photo-détecteurs convertissent la lumière captée en signaux électriques. Ils sont alignés sur la barrette parallèlement aux rangées des alvéoles et fonctionnent simultanément pour toutes les alvéoles de chaque rangée en cours dans le poste d'examen visiométrique. Si le flux de photons frappant un des photo-détecteurs est trop énergétique, le phénomène d'éblouissement précité apparaît, ce qui se traduit par une diffusion parasite d'électrons vers les éléments photo-détecteurs voisins. En conséquence la plus gênante dans l'industrie de la production de poussins, il s'ensuit par exemple que l'absence d'un oeuf dans une alvéole fausse le résultat pour les alvéoles voisines et que pour chacune de celles-ci, même si un oeuf y est présent on ne sait distinguer s'il est fécondé ou non. En d'autres termes, cet état de fait peut provoquer un dysfonctionnement de la chaîne de traitement des signaux (traitement effectué par le dispositif d'analyse automatique) et avoir pour résultat final d'empêcher une différenciation correcte entre les états de transparence présentés par les oeufs occupant les alvéoles appartenant à la même rangée que l'alvéole ayant provoqué l'erreur, qui sont examinées en même temps.

Dans l'état de la technique, tel qu'il ressort notamment des documents US 5 900 929, EP 1 457 108, US 3 540 824, US 4 843 958 et WO 00/36411, il est alors nécessaire de jeter tous les oeufs de cette rangée, ce qui conduit naturellement à un gâchis et à des pertes financières qu'il conviendrait d'éviter. Dans le cas d'un ensemble industriel, on ne peut envisager d'arrêter la procédure de détection, la cadence de tri étant très élevée, typiquement de l'ordre de 6 000 oeufs à l'heure.

On pourrait penser qu'il suffit de "contracter" l'échelle des intensités lumineuses pour éviter ce phénomène, en abaissant le niveau d'intensité lumineuse maximum (absence d'oeuf : intensité *I*₁). Cependant, on constate que la gamme *G*₃ est constituée de niveaux d'intensités lumineuses relativement proches les uns des autres. Il s'ensuit que la discrimination entre les trois niveaux de cette gamme est difficile à réaliser. Pour obtenir un bon contraste et être à même d'effectuer cette discrimination, il est nécessaire de recourir à une dose d'illumination relativement forte, ce qui a pour effet de dilater la gamme *G*₃, mais aussi corrélativement l'étendue de l'échelle complète des intensités, donc le niveau maximum d'illumination, et les risques d'éblouissement s'en trouvent aggravés. Dans un cas plus général, on peut rencontrer un nombre quelconque de gammes de niveaux d'intensités lumineuses très éloignées les unes des autres. Et comme dans le cas du mirage des oeufs, les échelles de sensibilité de l'examen sous rayonnement lumineux sont souvent plus proches d'une échelle de type logarithmique que d'une échelle proportionnelle.

Le besoin se fait donc sentir de pouvoir éviter les risques d'éblouissement du capteur de détection tout en conservant la possibilité de discriminations fines entre des intensités lumineuses de niveaux relativement proches les uns des autres, c'est-à-dire d'obtenir un bon contraste, ce qui paraît tout à fait antinomique.

L'invention vise à pallier les inconvénients des dispositifs de l'art connu. Elle propose à cette fin de réaliser l'examen visiométrique de chaque rangée en plusieurs étapes successives (au moins deux) en éclairant les emplacements à examiner par des doses d'illumination qui sont choisies différentes d'une étape à l'autre entre deux étapes successives et en n'éclairant dans une étape seconde que ceux des emplacements qui dans une étape première n'ont pas révélé présenter un état qui provoquerait un éblouissement du capteur dans l'étape seconde.

L'invention se traduit en particulier, en termes de procédé, par un procédé d'analyse d'objets maintenus en des emplacements respectant une répartition répétitive de lignes longitudinales et de rangées transversales sur un convoyeur les entraînant en défilement en direction longitudinale, à travers un poste d'examen visiométrique comportant des moyens capteurs sensibles à des faisceaux émergents retransmis desdits objets, caractérisé en ce que l'on réalise l'examen de chaque rangée en au moins deux étapes ou cycles de mesure successifs, en éclairant les emplacements à examiner par des doses d'illumination différentes et en n'éclairant dans une étape seconde que ceux des emplacements qui dans une étape première précédente n'ont pas révélé présenter un état qui provoquerait dans l'étape seconde un éblouissement des moyens capteurs pertubateur pour les emplacements voisins de la même rangée en cours d'examen.

Si l'on considère les cas d'application préférés de l'invention, où l'on retrouve des situations identiques ou similaires à celles du mirage des oeufs, c'est-à-dire où l'on vise à déterminer un état à attribuer à chacun desdits objets en fonction des conséquences qu'ils induisent sur un faisceau d'illumination auquel ils sont exposés, lors du passage de chacune des rangées successives d'un lot d'oeufs à trier suivant qu'ils sont clairs ou fécondés, ou d'objets similaires maintenus dans des alvéoles de casiers constituant lesdits emplacements, des caractéristiques secondaires de l'invention, s'appliquant individuellement ou simultanément dans toute combinaison techniquement opérante, sont avantageusement les suivantes :
Les deux cycles de mesure successifs sont alors avantageusement effectués respectivement au cours d'une étape seconde principale effectuée dans des conditions propres à déterminer un état de transparence ou similaire affectant l'atténuation du faisceau de manière sensible pour le capteur du faisceau émergent, qui est précédée d'une étape première effectuée dans des conditions propres à déceler la présence d'alvéoles vides et à déterminer et enregistrer les coordonnées de leurs emplacements dans la rangée en cours, afin de commander les conditions d'illumination lors de l'étape seconde pour ne pas les éclairer.

D'autre part, la dose d'irradiation adaptée pour la gamme de sensibilité de chaque étape est avantageusement réglée par variation du temps d'exposition sous une puissance émettrice déterminée. La dose d'irradiation étant choisie pour chaque étape afin d'éviter un risque d'éblouissement de détecteurs contigus autour d'un emplacement vide d'oeuf (ou objet similaire), on comprend aisément que la durée d'exposition des objets, pour chaque rangée, est relativement courte pour l'étape première et relativement longue pour l'étape seconde.

La source lumineuse éclairant les objets à examiner est avantageusement constituée d'une série de diodes électroluminescentes, ou "LED" selon l'abréviation anglo-saxonne couramment utilisée. Ces diodes ou LED sont disposées parallèlement aux rangées des emplacements à analyser, donc suivant une direction transversale au déplacement des objets entraînés par le convoyeur. Plus exactement, elles sont alignées parallèlement aux rangées d'alvéoles dans les casiers recevant les oeufs, donc en général suivant une rangée perpendiculaire à la direction longitudinale de convoyage.

Selon une caractéristique secondaire de l'invention, les doses d'illumination différentes sont appliquées lors d'au moins deux cycles successifs, suffisamment rapprochés dans le temps pour éclairer la même rangée en cours dans le poste d'examen visiométrique, qui utilisent les mêmes diodes sources, de la même intensité, mais pendant des durées différentes, de manière à ménager au moins deux gammes de sensibilité différentes, tout en évitant de l'une à l'autre le phénomène d'éblouissement de tout ou partie des photo-détecteurs.

Pendant le premier cycle de mesure, on applique une dose relativement faible et le dispositif d'analyse automatique détecte la présence ou non d'alvéoles vides, et s'il en trouve il détermine et mémorise leurs coordonnées dans la rangée examinée (numéro d'ordre en position transversale dans cette rangée). Pendant le deuxième cycle de mesure, on commande avec une dose relativement forte l'illumination des seuls emplacements d'alvéoles non vides. Le dispositif d'analyse automatique discrimine, si elles existent, les alvéoles contenant des d'oeufs clairs par distinction avec celles contenant d'autres catégories d'oeufs, notamment des oeufs fécondés, et il enregistre les coordonnées respectives de ces deux catégories potentielles d'alvéoles. Ces coordonnées sont particulièrement simples à exprimer par le numéro d'ordre de l'alvéole dans sa rangée et le numéro d'ordre de cette rangée en ligne longitudinale dans le casier.

Les doses lumineuses respectivement relativement faible et relativement forte sont choisies, la seconde pour permettre un contraste au niveau des photo-détecteurs distinguant les oeufs fécondés des oeufs non fécondés, la première pour déceler les emplacements où l'application de la seconde entraînerait pour les photodétecteurs correspondants la présence d'une intensité lumineuse trop élevée, susceptible d'induire un phénomène d'éblouissement.

D'autres caractéristiques de l'invention concernent l'organisation de dispositions spatiales corrélées pour les photo-détecteurs de la caméra et les diodes d'illumination, l'activation de ces deux séries de composants étant synchronisée sous la commande de moyens automatiques.

Ainsi, dans le cas où les alvéoles présentent une configuration en quinconce, il est avantageux de grouper les rangées d'alvéoles deux par deux en prévoyant une source lumineuse constituée d'un nombre de LED double de celui du nombre d'alvéoles par rangée. Dans cette configuration chaque diode est activée en correspondance avec le passage dans son champ d'une rangée d'alvéoles sur deux. En d'autres termes, dans ce mode de réalisation, la moitié des diodes sont affectées aux lignes d'alvéoles de rang pair, l'autre moitié aux lignes de rang impair.

On peut également multiplier le nombre de cycles d'illumination, chaque cycle impliquant une dose d'illumination différente (notamment par une durée propre pour une intensité lumineuse restant identique), par exemple procéder à trois cycles. Lors du troisième cycle, dans l'application préférée de l'invention concernant le mirage des oeufs, le dispositif d'analyse automatique différencie les oeufs réellement fécondés des autres catégories d'oeufs non clairs présents (oeufs à faux germe, oeuf pourri). Seules les alvéoles susceptibles de contenir l'une ou l'autre de ces catégories d'oeufs non clairs (suite à l'analyse effectuée lors du deuxième cycle de mesure) sont éclairées lors du troisième cycle.

Suite à ces discriminations successives, les oeufs peuvent être triés et/ou marqués en sortie de l'installation de mirage des oeufs. Dans la pratique et dans un mode de réalisation préféré, ce sont les oeufs clairs qui sont marqués et/ou triés, pour être éliminés, seuls les oeufs fécondés étant conservés. En général on se contente donc de procéder en deux étapes, dans des conditions opératoires qui sont déterminées de manière à ne pas éclairer les alvéoles vides lors de la seconde étape d'illumination permettant de détecter la présence d'oeufs clairs, non fécondés, en considérant qu'il importe peu qu'il subsiste avec les oeufs correctement fécondés des oeufs à faux germe ou même des oeufs pourris, qui ne pourront ni les uns ni les autres voir naître un poussin.

Toutefois, il peut également être utile de procéder à une analyse plus poussée en opérant en plus de deux étapes. En particulier l'invention permet de dresser des statistiques utiles en enregistrant les résultats dans des fichiers informatiques et en les soumettant à des calculs adaptés pour déterminer des données telles que la rentabilité de la fécondation ou la qualité d'une livraison reçue dans une écloserie.

En termes de dispositif, l'invention se traduit notamment par un système d'analyse opto-électronique s'appliquant préférentiellement à une installation de mirage d'oeufs maintenus dans des alvéoles de casier qui sont susceptibles de présenter au moins deux états, fécondé et clair respectivement, lesdites alvéoles étant disposées en des emplacements respectant une configuration déterminée de lignes et de rangées.

Ladite installation comporte de manière connue un dispositif de convoyage entraînant des casiers d'oeufs successifs à une vitesse déterminée à travers un poste d'examen visiométrique qui comporte une source lumineuse, générant un faisceau d'illumination propre à chacun desdits oeufs de chaque rangée successivement en cours dans ledit poste, et des moyens de détection synchronisée des faisceaux émergeant desdits oeufs, ainsi que des moyens déterminant automatiquement un état desdits oeufs, notamment un état fécondé ou non, en fonction de l'atténuation induite par chaque oeuf sur le faisceau correspondant.

Dans les modes de mise en oeuvre de l'invention les mieux adaptés à la pratique industrielle, la source lumineuse est constituée d'une pluralité de diodes électroluminescentes alignées parallèlement aux rangées d'emplacements de réception d'oeufs dans les casiers (les alvéoles), et les moyens de détection sont réalisés sous la forme d'un capteur constitué d'une barrette d'une pluralité de photo-détecteurs sensibles à la lumière émise, dont la configuration spatiale est corrélée avec celle desdites diodes électroluminescentes. Des moyens appropriés sont alors prévus pour piloter en synchronisme d'une part l'émission sélective de faisceaux par lesdites diodes électroluminescentes, de manière à éclairer simultanément des alvéoles prédéterminées dans chaque rangée en cours dans le poste d'examen, et d'autre part la réception des faisceaux émergents par des photo-détecteurs dudit capteur en relation spatiale avec les diodes électroluminescentes émettant de la lumière.

Suivant l'invention, un tel pilotage est programmé pour assurer automatiquement au moins deux cycles de mesure d'atténuation par émission de lumière de durées prédéterminées délivrant des doses d'illuminations différentes, chacune évitant un éblouissement des moyens de détection sensibles aux faisceaux émergents, à savoir un premier cycle au cours duquel la lumière émise éclaire toutes les alvéoles de la rangée en cours pendant une première durée, de manière à déterminer l'existence ou non d'alvéoles vides d'oeuf, se traduisant par la détection d'une lumière non atténuée à haute énergie, et à enregistrer dans des moyens de mémoire les coordonnées des emplacements suivant que les alvéoles correspondantes sont ou non vides d'oeuf, et un second cycle au cours duquel la lumière émise éclaire les seules alvéoles dont les coordonnées indiquent qu'un oeuf y est présent, pendant une deuxième durée, plus longue que ladite première durée, de manière à discriminer les oeufs fécondés des oeufs clairs, par détection d'atténuations différentes de la lumière émergeant desdits oeufs. Pour exploitation ultérieure, éventuellement dans un poste suivant de la même installation, des moyens sont prévus pour la mise en mémoire des coordonnées des oeufs fécondés et/ou des oeufs clairs.

L'invention va maintenant être décrite de façon plus détaillée en se référant aux dessins annexés, parmi lesquels :
- la figure 1 illustre schématiquement un exemple d'une installation de mirage des oeufs incorporant un poste d'examen visiométrique ainsi qu'un poste de marquage des oeufs non fécondés ;
- la figure 2 illustre schématiquement un mode de configuration préféré pour un casier de transport des oeufs à examiner que l'on introduit dans l'installation de la figure 1, en vue partielle de dessus ;
- la figure 3 illustre schématiquement une échelle des intensités lumineuses des faisceaux émergents pour diverses catégories d'oeufs lorsqu'ils sont illuminés par une source de lumière infrarouge ;
- la figure 4 illustre schématiquement une configuration de source lumineuse à LED mise en oeuvre dans le système de détection et d'analyse automatique de la figure 1 ;
- et la figure 5 est une figure de détail illustrant l'illumination d'un oeuf et la détection par un capteur de la lumière retransmise depuis cet oeuf.

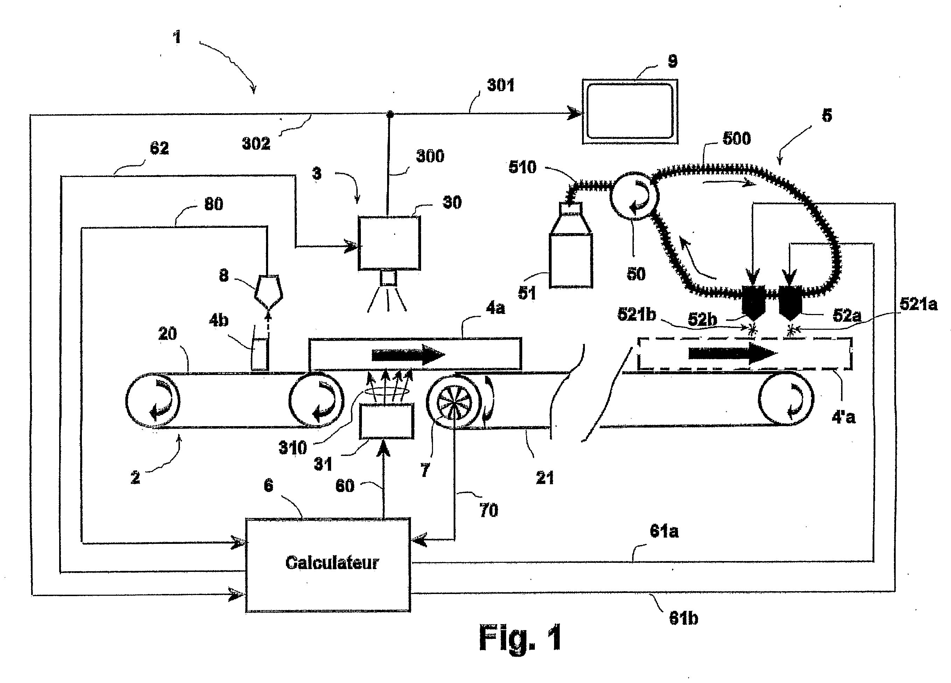

Avant de décrire, par référence à la figure 1, le fonctionnement du système optoélectronique comprenant un dispositif de détection et un dispositif d'analyse automatique d'oeufs fécondés proprement dit, on va décrire un exemple d'architecture d'une installation de mirage des oeufs 1 incorporant un tel système, selon un mode de réalisation préféré de l'invention. Dans les figures qui suivent, les éléments communs portent les mêmes références et ils ne seront re-décrits qu'en tant que de besoin.

A l'exception des caractéristiques avantageuses spécifiques à l'invention qui seront signalées et détaillées ci-après, l'architecture générale d'une telle installation est en grand partie commune, en soi, à celles des installations de l'art connu. On pourra ici se reporter au brevet français publié sous le numéro 2 768 517 par exemple. C'est d'ailleurs un avantage supplémentaire du système de l'invention, qui permet de ré-utiliser des technologies bien connues et des matériels financièrement amortis.

Ainsi, l'installation 1 comprend un convoyeur 2, à tapis circulant en boucle fermée ou dispositif équivalent, présentant dans l'exemple illustré une portion d'entrée 20 et une portion de sortie 21. Ce convoyeur entraîne en translation à travers le poste d'examen visiométrique, en principe à vitesse constante, des casiers contenant les oeufs à mirer (non représentés), qui sont introduits l'un après l'autre, manuellement, à l'entrée de l'installation. De façon avantageuse, les casiers sont ceux ayant servi à l'incubation des oeufs.

Un poste d'examen visiométrique 3 est disposé fixe entre les deux portions de convoyeur 20 et 21. Il comporte, en partie inférieure une source de rayonnement 31, qui dans le cas particulier considéré émet des faisceaux individuels de lumière infrarouge, et en partie supérieure des moyens de détection, qui sont sensibles à la longueur d'onde de la lumière émise par la source 31 et qui sont constitués par des détecteurs discrets ou, de façon préférentielle, par une caméra vidéo 30. La source 31 comme le capteur de la caméra 30 seront plus complètement décrits plus loin, dans leur constitution et dans leur fonctionnement synchronisé, en liaison avec les figures 4 et 5.

Chacun des casiers d'oeufs introduits successivement sur le convoyeur 2, par exemple le casier 4a, coupe le faisceau 310 émis par la source 31 entre les deux portions de convoyeur. Il comporte une pluralité d'alvéoles dans lesquelles sont placés les oeufs à mirer. La structure de ces alvéoles est telle qu'elles laissent passer le faisceau 310 en l'absence d'oeufs (le fond est évidé en général). Les oeufs contenus dans ces alvéoles interceptent le faisceau 310 et le retransmettent avec une atténuation variable en fonction de leur état, et notamment, dans le cadre de l'application décrite, en fonction de leur état de fécondation, suivant que l'oeuf est à l'état fécondé ou non. Cet effet à mesurer n'est pas dû à proprement parler à la qualité de transparence plus ou moins bonne de l'oeuf, mais plutôt à une diffusion plus ou moins importante de la lumière qui pénètre dans l'oeuf. C'est pourquoi d'ailleurs la mesure implique que le faisceau d'illumination frappe la coquille de l'oeuf correspondant, même s'il n'est pas précisément suivant son axe.

Le rayonnement reçu par la caméra 30 est converti en signaux électriques qui sont transmis sur une liaison de sortie 300, de façon avantageuse sous forme de signaux numériques, d'une part à un organe de visualisation 9, par exemple un écran cathodique (liaison 301), d'autre part à un système de traitement automatique de données à programme enregistré 6 (liaison 302), que l'on appellera ci-après simplement calculateur. Ce dernier peut être un processeur de signaux numériques dédié, ou être d'un type standard et muni de ports appropriés. Le calculateur analyse, par traitement d'image, sous la conduite d'un logiciel spécifique et de façon connue en soi, les signaux d'images reçus de la caméra 30. Le traitement d'image ainsi réalisé permet de déterminer si les oeufs analysés sont ou non fécondés, en situant le taux d'atténuation du faisceau lumineux à la traversée de chaque oeuf par rapport à des valeurs seuils limitant des gammes prédéterminées.

Dans une variante de réalisation de l'installation décrite, l'organe de visualisation 9, qui est optionnel, peut être piloté par des signaux reçus du calculateur 6, et non directement de la caméra 30, c'est-à-dire après traitement des signaux.

Un mode de réalisation préféré des casiers d'oeufs mis en oeuvre dans les conditions d'application pratique de l'invention, par exemple le casier 4a, est illustré schématiquement par la figure de détail 2 (en vue partielle de dessus). On y voit une pluralité d'alvéoles, sous les références générales 40 à 44, qui sont destinées à recevoir les oeufs à mirer (non expressément représentés). Ces alvéoles, 40 à 44, sont disposées en rangées successives, orientées parallèlement entre elles transversalement à la direction longitudinale de déplacement (flèche *f*). La figure représente ainsi cinq rangées *R*₀ à *R*₄ sur quatre lignes 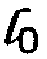 à 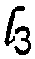 dans la direction longitudinale, dans une disposition qui respecte une matrice orthonormée. Toutefois, d'une rangée à la suivante les alvéoles se disposent en quinconce. Dans une disposition plus précisément à maille hexagonale, elles sont décalées d'un demi-pas en direction transversale entre les rangées d'ordre pair et les rangées d'ordre impair.

La source infrarouge 31 (figure 1) est composée d'une pluralité de diodes électroluminescentes ou LED, selon l'abréviation anglo-saxonne (pour "Light Emitting Diode"). Ces diodes sont disposées sur une ligne parallèlement aux rangées, *R*₀ à *R*₄, des casiers, par exemple 4a, c'est-à-dire suivant une direction orthogonale au déplacement. Elles sont espacées les unes des autres, dans le cas particulier considéré ici de la valeur d'un demi-pas, de manière à parcourir chacune les alvéoles successives d'une même ligne respectivement, au cours du déplacement relatif. Les LED 31 sont commandées en mode impulsionnel par le calculateur 6 (liaison 60), à un rythme déterminé en fonction de la vitesse du convoyeur, et en synchronisme avec le défilement des alvéoles, afin que chaque diode produise un faisceau élémentaire d'illumination d'une alvéole au moment de son passage devant la diode, et que donc ledit faisceau se voit modifié par l'oeuf qu'elle contient avant d'être détecté par la caméra 30 pour être analysé.

La figure 4 illustre schématiquement la configuration de la source de lumière 31 de la figure 1, qui est composée d'une pluralité de diodes électroluminescentes ou "LED" selon l'abréviation anglo-saxonne (pour "Light Emitting Diode"), émettant dans l'infrarouge. Ces diodes sont disposées en ligne parallèlement aux rangées des casiers, donc à une rangée *Rₓ* d'ordre x supposée en cours d'examen, c'est-à-dire suivant une direction transversale orthogonale à la direction longitudinale de défilement à travers le poste d'examen visiométrique.

Dans le cas particulier illustré pour une configuration d'alvéoles en quinconce, le nombre de diodes, *D*_{*X*1} à *D*_{*X*4}, est double de celui des alvéoles d'une rangée. On a supposé que la rangée *Rₓ* était d'ordre impair et comprenait les alvéoles 4*x*1 et 4*x*3 (en supposant qu'il y ait quatre lignes), symbolisées par des ellipses en pointillés. Les diodes ont été référencées *D*_{*X*1} à *D*_{X4}. Dans l'exemple décrit, à l'instant représenté sur la figure 4, seules les diodes *D*_{*X*1} et *D*_{X3} sont activées pour les rangées d'ordre impair, car situées sous les alvéoles 4*x*1 et 4*x*3. Lorsque la rangée d'alvéoles suivante se trouvera au-dessus des diodes, ce seront les diodes *D*_{*X*2} et *D*_{X4} qui seront activées pour cette rangée d'ordre pair.

Il importe toutefois de souligner que cette disposition n'est nullement limitative des conditions de mise en oeuvre de l'invention. Il existe bien des situations où l'on aura plutôt intérêt à réaliser l'éclairage au moyen de plusieurs groupes de diodes, notamment deux ou trois groupes implantés l'un à côté de l'autre. On augmente ainsi la polyvalence de la machine, qui peut s'adapter facilement à des casiers de dimensions et pas différents. L'allumage des diodes est commandé de manière sélective en fonction de la disposition des alvéoles dans les casiers. La sélection des diodes à allumer est fonctionnellement équivalente à l'ajustage mécanique de la position des diodes sous les alvéoles.

Dans tous les cas chaque diode allumée produit un faisceau élémentaire destiné à illuminer individuellement l'une des alvéoles de la rangée en cours d'examen dans le poste de visiométrie. Les diodes dans leur ensemble sont commandées en mode impulsionnel par le calculateur 6 : liaison multiple 60.

La figure 5 illustre schématiquement l'illumination d'un oeuf *OX*1, disposé dans l'alvéole 4*x*1 de la rangée *R_{X}* par la diode *D*_{*X*1}.

La caméra 30 comprend un capteur référencé *CCD* constitué par une barrette de photo-détecteurs du type "CCD" précité. Selon une caractéristique importante de l'invention, le capteur *CCD* est piloté par le calculateur 6 en synchronisme avec le pilotage des diodes, *D*_{*X*1} à *D*_{X4}. En outre la configuration spatiale linéaire de ce capteur est corrélée à celle de ces diodes. Le pilotage du capteur *CCD* est obtenu par la génération de signaux de commande sur une liaison 62 reliant le calculateur à une entrée de commande de la caméra 30.

Si l'on se reporte au diagramme de la figure 3, l'illumination sans précaution d'une alvéole vide d'oeufs risque de provoquer un éblouissement de photo-détecteurs du capteur *CCD* recevant le flux lumineux qui n'a subi aucune atténuation. L'intensité lumineuse captée *l*₁ est en effet très élevée. Pour fixer les idées, si la caméra utilisée supporte un courant moyen de 100 mA (après conversion de l'énergie lumineuse en signaux électriques), une impulsion lumineuse induisant un courant de 1 A, si sa durée est suffisante, va générer un courant moyen dépassant la limite admise de 100 mA. Le phénomène d'éblouissement va donc se produire.

Aussi, selon une caractéristique importante de l'invention, on prévoit d'appliquer deux cycles de mesure à chaque rangée successivement en cours d'examen.

Le premier cycle consiste à générer, sous la commande du calculateur (liaison 60), une impulsion de lumière illuminant chacune de toutes les alvéoles de la rangée. Les signaux impulsionnels de commande sont transmis à toutes les diodes, *D*₁₁ à *D*₂₄. Toujours à titre d'exemple, la durée de cette impulsion est typiquement de l'ordre de 100 µs, pour les caractéristiques de caméra indiquées ci-dessus.

Ce premier cycle de mesure permet de détecter les emplacements éventuels d'alvéoles vides d'oeuf. Le calculateur 6 autorise l'activation (signal de commande sur la liaison 62) des photo-détecteurs du capteur *CCD* situés sur les lignes des alvéoles de la rangée en cours, reçoit (liaison 302) les signaux électriques issus de la conversion opto-électronique effectuée par ce capteur, analyse les signaux d'image ainsi reçus et les soumet à un traitement automatique au terme duquel il commande l'enregistrement, dans des moyens de mémoire (non représentés) qui lui sont associés, des coordonnées dans le casier en cours des alvéoles vides dont l'existence a été détectée, par distinction avec les alvéoles dans lesquelles un oeuf est présent.

Ensuite, un deuxième cycle de mesure est initié. En tout ou partie, les diodes *D*_{*X*1} à *D*_{X4} reçoivent une deuxième impulsion de commande générée par le calculateur 6 pour éclairer de nouveau les oeufs présents dans leurs alvéoles, par exemple l'oeuf *OX*1. L'illumination est sélective. Seules les diodes en relation de correspondance spatiale avec des alvéoles non vides sont activées. Sur la liaison 60 le calculateur 6 ne transmet donc des signaux de commande qu'à ces diodes, ce en fonction des résultats d'analyse obtenus à l'issue du cycle précédent et des coordonnées enregistrées distinguant les alvéoles vides et non vides. La durée de l'impulsion est plus élevée que celle de la première impulsion, de manière à exposer les oeufs à une quantité de lumière plus importante, étant donné que l'on opère avec une intensité lumineuse identique. De façon synchronisée, le calculateur 6 envoie un signal (liaison 62) à la caméra autorisant la détection des faisceaux émis par les diodes activées tels qu'ils sont retransmis atténués par les oeufs.

Toujours à titre d'exemple, la durée de l'impulsion générée pendant le deuxième cycle est typiquement de l'ordre de 1 ms. Ce temps d'exposition permet de différencier les oeufs clairs (figure 3 : intensité *I*₂) des autres catégories d'oeufs, les intensités lumineuses (*I*₃ à *I*₅) transmises au travers des oeufs et reçues par le capteur *CCD* pour ces catégories étant proches les unes des autres. Cette différenciation est effectuée par le calculateur 6, qui reçoit à cet effet les signaux (liaison 302) issus de la conversion opto-électronique réalisée par le capteur.

Puisque les alvéoles vides (si elles existent) ne sont pas exposées, il n'y a plus de risque d'éblouir des photo-détecteurs, l'atténuation apportée par les autres catégories d'oeufs, quelles qu'elles soient, étant suffisamment forte.

Pour chaque rangée en cours les deux cycles se succèdent à une cadence suffisamment rapide pour que les axes de symétrie verticale Δ (figure 5) des oeufs illuminés n'aient pas le temps de se mouvoir de façon significative en regard des conditions de l'examen, compte tenu de la vitesse de défilement qui leur est imprimée par le convoyeur 2 (figure 1) par translation relative par rapport à l'équipement d'émission des faisceaux incidents et de détection des faisceaux émergents. On assure ainsi que les faisceaux émis successivement d'un cycle à l'autre frappent correctement les mêmes oeufs. Ceci est illustré sur la figure 5 en admettant que les faisceaux traversent l'oeuf *OX*1 pour ressortir dans des zones très proches les unes des autres, à l'intérieur d'une zone sensiblement circulaire Z*s* de faible dimension rayon autour du sommet de l'oeuf. Cette condition est aisée à remplir, car la vitesse de translation du convoyeur est faible comparée aux vitesses que l'on peut atteindre dans le domaine de l'opto-électronique.

Toujours pour fixer les idées, si l'on considère un rythme de défilement typiquement de 36 000 oeufs par heure, chaque rangée contenant 6 oeufs, et un pas entre alvéoles de 40 mm (de façon plus générale ce pas est compris entre 30 et 50 mm), le temps de passage sous la caméra 30 est d'environ 600 ms. Compte tenu de la technologie disponible pour des applications de ce type, on peut estimer qu'un temps de 150 ms, environ, est amplement suffisant pour effectuer la saisie des images par la caméra 30, et l'analyse et le traitement des signaux d'image reçus par le calculateur 6. Durant ce laps de temps, le sommet de l'oeuf n'aura avancé que de 10 mm, soit de ± 5 mm par rapport à l'axe. Des écarts doubles ou triples sont possibles tout en conservant une précision suffisante dès lors que l'essentiel n'est pas que le faisceau traverse l'oeuf suivant son diamètre, mais qu'il frappe la sphère inférieure de la coquille. D'où la possibilité de soumettre chaque rangée d'alvéoles à un troisième cycle de mesure, éventuellement un quatrième, en augmentant à chaque fois la durée d'exposition et en excluant ceux des emplacements qui à l'étape précédentes, dite étape première, ont révélé pour l'oeuf correspondant un état qui provoquerait à l'étape suivante (étape seconde) un éblouissement du capteur.

En particulier, cette possibilité peut avantageusement être mise à profit pour obtenir une discrimination supplémentaire entre les catégories d'oeufs à l'intérieur de la gamme *G*₃ (figure 3 : oeufs pourris, oeufs réellement fécondés et oeufs contenant un faux germes). On procède alors à un troisième cycle de mesure, de durée différente des deux premiers. Toujours pour fixer les idées, les durées respective des trois cycles pourraient être typiquement les suivantes : 100 µs, 1 ms et 4 ms.

Le déroulement des deux premiers cycles est tout à fait semblable à ce qui vient d'être décrit pour un processus à deux cycles seulement. A l'issue des deux premiers cycles, une discrimination a pu être effectuée entre les alvéoles vides (premier cycle) et entre, d'une part les oeufs clairs, et d'autre part les autres catégories d'oeufs (deuxième cycle). Les coordonnées des catégories d'oeufs ainsi discriminées à l'issue du deuxième cycle sont enregistrées par le calculateur 6 dans des moyens de mémorisation.

Lors du troisième cycle, les alvéoles susceptibles de contenir des oeufs dans la gamme *G*₃ (figure 3) sont illuminées par la troisième impulsion. Le mode opératoire est semblable à celui du deuxième cycle. Le calculateur 6 envoie des signaux de commande synchronisés à la caméra 30 et aux seules diodes qui se trouvent en vis-à-vis d'alvéoles susceptibles de contenir les oeufs dans un état conduisant à une atténuation de la gamme *G*₃. De ce fait, il devient possible de différencier ces catégories d'oeufs. Une application intéressante consiste à dénombrer séparément chacune des catégories ainsi distinguées, ce qui fournit un outil d'évaluation de la qualité de la fécondation chez l'incubateur, du taux de remplissage des casiers, du rendement à attendre de l'éclosoir.

Après analyse du contenu des casiers et de la mise en mémoire des coordonnées des diverses catégories discriminées, deux au minimum, à savoir oeufs clairs et oeufs fécondés (ou portant un faux germe, ou pourris), voire un plus grand nombre de catégories (processus à trois cycles, ou plus), ces casiers continuent leur cheminement à l'intérieur de l'installation de mirage, entraînés par le convoyeur 2, jusqu'à ressortir de cette installation 1.

De façon pratique, il existe alors trois possibilités principales (qui peuvent se cumuler) :
- marquage des oeufs selon une seule classe ou plusieurs classes ;
- simple tri ;
- constitution de statistiques enregistrées dans des fichiers informatiques, affichées et/ou imprimées sur des listings.

Il est généralement souhaitable de marquer au moins les oeufs clairs, non fécondés, qui doivent être écartés de la ligne conduisant à l'éclosoir pour la production de poussins. Dans la pratique, après marquage, ils sont éliminés manuellement en sortie de l'installation, éventuellement pour être récupérés. Ils peuvent servir en alimentation ou servir de milieu de culture pour la fabrication de vaccins.

Pour marquer sélectivement les oeufs en fonction de la catégorie dont ils relèvent, connaissant leurs coordonnées dans les casiers, mais cette connaissance n'est pas suffisante, on prévoit de corréler temporellement la sortie d'un oeuf d'une catégorie donnée, que l'on désire marquer, avec l'instant du marquage, qui est effectué lors du passage des oeufs, rangée par rangée, dans une zone prédéterminée de sortie de l'installation, sous un dispositif de marquage. Pour ce faire, si on considère de nouveau la figure 1, on prévoit un organe 8, de tout type approprié, détectant le début du passage d'un nouveau casier d'oeufs à mirer sur le convoyeur 2, par exemple le casier 4b, et qui délivre sur une liaison de sortie 80 une impulsion de synchronisation transmise au calculateur 6. De façon préférentielle également, le convoyeur 2 comprend un capteur de déplacement 7 délivrant, sur une liaison de sortie 70, des signaux permettant de déterminer l'amplitude du déplacement de ce convoyeur 2. Ces signaux, corrélés avec l'instant d'émission de l'impulsion de synchronisation (liaison 80) permettent le calcul, à tout instant, de la position atteinte par un casier donné. De cette façon, il est notamment possible de connaître avec précision l'instant de sortie d'un casier, par exemple le casier 4a : référencé 4'a lorsqu'il sort de l'installation 1 après avoir parcouru toute la longueur de la portion de sortie 21 du convoyeur 2.

De façon spécifique dans le cas particulier d'application décrit pour illustrer la mise en oeuvre de l'invention, le système de marquage 5 selon l'invention est essentiellement constitué par une pluralité de dispositifs encreurs à organes éjecteurs d'encre, ou buses. Ces dispositifs sont montés fixes au-dessus du convoyeur. En correspondance avec la disposition en quinconce des alvéoles des casiers, ils sont répartis en deux sous-ensembles 52a et 52b, dans une disposition également en quinconce entre deux rangées parallèles d'autant de dispositifs encreurs qu'il y a d'alvéoles dans une rangée de casier. Dans la direction transversale, l'écartement entre les dispositifs encreurs est égal à un pas de la répartition des alvéoles, ce sur chacune des deux rangées. Dans la direction longitudinale l'écart entre les deux rangées est avantageusement d'un demi-pas comme pour les alvéoles, ce qui permet de commander tous les dispositifs encreurs en même temps. Cependant, on peut aussi procéder différemment, quand par exemple il est souhaitable d'écarter plus les deux sous-ensembles l'un de l'autre, en utilisant de façon concomitante une vitesse de défilement choisie plus lente dans le poste de marquage que dans le poste d'examen optique.

Chaque dispositif encreur est construit à l'identique d'un dispositif injecteur d'essence tel que les injecteurs utilisés par ailleurs dans l'industrie automobile pour alimenter en carburant les cylindres d'un moteur à explosion. Les injecteurs sont alimentés par une pompe 50, via des conduits constitués, par exemple, de tuyaux souples en matériau synthétique, branchés sur un même circuit en boucle 500 qui est alimenté à partir d'un réservoir de liquide colorant 51, via un conduit 510, de manière à maintenir une pression constante de liquide dans une chambre tampon propre à chaque injecteur. Le liquide colorant est non aqueux, pour éviter les risques de rouille, les différents organes du circuit, injecteurs et pompes, étant réalisés à base d'acier. De façon préférentielle, on utilise un produit colorant en milieu alcoolique. Pour un colorant soluble ou un pigment insoluble en dispersion, l'alcool courant a le double avantage d'être un solvant organique aisément disponible et peu coûteux et d'être compatible avec un usage alimentaire.

Les ordres de commande des injecteurs de marquage sont délivrés par le calculateur 6, sous la forme d'impulsions qui sont transmises sur deux séries de liaisons, 61a et 61b, et qui, pour chaque injecteur commandé en marquage, sont adressés à une valve électromagnétique qui détermine l'ouverture de la buse libérant le liquide colorant, provoquant ainsi l'émission d'un jet d'encre sous pression, 521a ou 521b, qui va marquer l'oeuf passant sous la buse correspondante à cet instant.

Le système de marquage ainsi utilisé suivant l'invention est particulièrement bien adapté du fait que les marques à apposer sur les oeufs sont des marques simples, qui représentent des taches élémentaires et qui ne sont pas nécessairement à préserver dans le temps, et d'autre part le marquage à effectuer ne vise pas tous les objets qui défilent dans l'installation, mais seulement certains d'entre eux préalablement identifiés (oeufs non fécondés notamment). Les besoins sont donc radicalement différents de ceux qui prévalent, par exemple, quand il s'agit de marquer des oeufs en vue de préciser des informations destinées aux consommateurs, telles que la date de ponte ou des informations similaires, auquel cas on doit avoir recours à des technologies d'impression sophistiquées pour composer chaque caractère à partir d'une matrice de pixels.

Dans leur montage mécanique les dispositifs encreurs, avec leurs buses respectives, sont immobiles, avantageusement fixés suivant les lignes de défilement des objets sur des barres supports transversales à la direction de déplacement, dans une disposition qui les affecte chacune à l'un respectivement des objets d'une même rangée passant à leur niveau. Le liquide encreur y est en permanence disponible, sous une pression suffisante pour que le jet d'encre atteigne l'objet à marquer. Son expulsion, pour chaque buse individuellement, est déclenchée par ouverture d'une valve au moment du passage d'un objet à marquer. L'absence de contact du dispositif encreur lui-même avec l'objet évite de risquer une détérioration de celui-ci, si bien que dans le cas des oeufs par exemple, on n'a pas à craindre de briser leur coque.

Dans des modes de réalisation préférés du système de marquage suivant l'invention, les dispositifs encreurs sont montés sur une ou plusieurs barres formant supports qui sont disposées au-dessus du plan parcouru par les objets et orientées parallèles aux rangées de leur répartition (direction transversale), l'écart entre deux injecteurs, ou pas, étant corrélé au pas des emplacements des objets, donc notamment au pas des alvéoles des casiers dans le cas du mirage des oeufs.

Dans un mode de réalisation particulier suivant l'invention, les injecteurs d'une barre sont assujettis à leur support par des moyens d'accrochage non permanents permettant un verrouillage/déverrouillage aisé et un réglage de la position de chaque injecteur le long du support. Par cette caractéristique, où les injecteurs relevant d'une même rangée sont montés sur leur barre support dans des positions réglables latéralement, le dispositif de marquage peut facilement accommoder diverses configurations de casiers contenant les objets à marquer, oeufs, fruits, ou autres. On peut ainsi, notamment, modifier le pas d'écartement entre deux injecteurs adjacents dans des dispositions qui peuvent ou non conserver l'équidistance entre les emplacements dans chaque rangée.

Dans des modes de mise en oeuvre de l'invention s'appliquant avantageusement aux situations où les casiers présentent une configuration en quinconce des alvéoles, le dispositif de marquage comprend, comme il est décrit ci-dessus, deux barres supports parallèles, les injecteurs d'une barre étant décalés latéralement par rapport aux injecteurs de l'autre barre en corrélation spatiale avec la configuration en quinconce des objets. Dans d'autres formes de mise en oeuvre de l'invention s'utilisant en variante, on réalise le montage des injecteurs sur leur barre support commune de manière à pouvoir les déplacer latéralement d'une distance correspondant à l'écart entre les objets d'une rangée à la suivante, et en fonctionnement, on commande le déplacement latéral en correspondance avec le passage des rangées successives.

La commande de marquage s'effectue en correspondance avec la détermination de l'état fécondé ou non des oeufs, en tenant compte de leurs positions latérales dans une rangée particulière d'alvéoles du casier et du temps nécessaire à cette rangée d'alvéoles pour parcourir la distance séparant leur position lors de l'examen détectant leur état de leur arrivée devant les buses d'impression correspondant aux alvéoles recevant les oeufs à marquer. Autrement dit, dans une installation intégrant le système de marquage en aval d'un système de mirage, les opérations de marquage s'effectuent au même rythme que les opérations d'examen visiométrique avec un décalage dans le temps qui est réglé par la vitesse du convoyeur faisant défiler les casiers d'oeufs.

## Revendications

1. Procédé d'analyse d'objets maintenus en des emplacements respectant une répartition répétitive de lignes longitudinales et de rangées transversales sur un convoyeur les entraînant en défilement en direction longitudinale à travers un poste d'examen visiométrique comportant des moyens d'illumination éclairant lesdits emplacements dans chaque rangée en cours d'examen par des faisceaux incidents individuels et des moyens capteurs sensibles aux faisceaux émergents retransmis desdits objets, ainsi que des moyens d'analyse par mesure d'atténuation entre faisceau incident et faisceau émergent pour chaque emplacement,
dans lequel lesdits objets sont des oeufs individuellement maintenus dans des alvéoles de casiers constituant lesdits emplacements, le procédé comportant l'examen de chaque rangée en au moins deux étapes de cycles de mesure successifs, en éclairant les alvéoles à examiner par des doses d'illumination différentes,
dans lequel dans la première étape, on éclaire toutes les alvéoles de la rangée en cours dans des conditions propres à déceler la présence d'alvéoles vides et on détermine et enregistre en mémoire les coordonnées de leurs emplacements dans ladite rangée en cours et,
dans la seconde étape, on éclaire les seules alvéoles non vides de ladite rangée dans des conditions propres à déterminer un état de chacun desdits oeufs par mesure d'atténuation entre faisceau incident et faisceau émergent pour chaque alvéole.

2. Procédé suivant la revendication 1, **caractérisé en ce que** la dose d'illumination appliquée à chaque étape est réglée par variation du temps d'exposition sous une puissance lumineuse déterminée, la durée d'exposition étant plus brève pour la première étape et plus longue pour la seconde étape.

3. Procédé suivant la revendication 1 ou 2 suivant lequel les conditions d'illumination respectives des deux étapes sont choisies pour éviter un éblouissement des moyens capteurs qui perturberait l'analyse en opérant dans deux gammes de sensibilité différentes permettant respectivement, dans ladite première étape de distinguer les alvéoles vides d'oeufs de celles dans lesquelles un oeuf est présent, qu'il soit fécondé ou non, et dans ladite seconde étape de distinguer les alvéoles dans lesquelles l'oeuf présent est fécondé et celles dans lesquelles l'oeuf présent n'est pas fécondé.

4. Installation (1) pour le mirage des oeufs, comportant
des casiers (4a) à alvéoles recevant les oeufs à analyser en des emplacements disposés suivant une configuration répétitive de lignes longitudinales et de rangées transversales,
un convoyeur (2) d'entraînement en direction longitudinale desdits casiers, pour les faire défiler à travers un poste d'examen visiométrique (5),
le poste d'examen visiométrique (3) comportant
une source lumineuse (31) générant un faisceau incident d'illumination de chacun desdits oeufs de chaque rangée successivement en cours dans le poste d'examen visiométrique et
des moyens capteurs de détection (30) des faisceaux émergents retransmis par lesdits oeufs,
ainsi que des moyens d'analyse automatique par mesure de l'atténuation subie par la lumière entre faisceau incident et faisceau émergent en chaque emplacement d'alvéole,
des moyens de pilotage (6) commandant en synchronisme ladite source lumineuse (31) et lesdits moyens de détection (30) pour soumettre chaque rangée d'alvéoles à au moins deux cycles de mesure successifs sous des doses d'illumination différentes, à savoir :
un premier cycle au cours duquel on éclaire toutes les alvéoles de la rangée en cours en appliquant une dose d'illumination relativement faible, on détermine en fonction de l'atténuation de la lumière entre faisceau incident et faisceau émergent pour chaque alvéole s'il s'agit ou non d'une alvéole vide, ne contenant pas d'oeuf, et l'on enregistre en mémoire les coordonnées de toute alvéole vide dont la présence est ainsi détectée dans ladite rangée,
et un deuxième cycle au cours duquel, en appliquant une dose d'illumination relativement forte, on éclaire les seules alvéoles de la rangée en cours dont les coordonnées indiquent qu'un oeuf y est présent, dans des conditions propres à déterminer automatiquement un état de chacun desdits oeufs en fonction de l'atténuation subie par la lumière entre faisceau incident et faisceau émergent pour chaque alvéole.

5. Installation suivant la revendication 4, configurée pour discriminer lesdits oeufs suivant des états fécondés et clairs en fonction de l'atténuation de l'intensité lumineuse induite par chaque oeuf entre faisceau incident et faisceau émergent telle que déterminée au cours dudit second cycle de mesure.

6. Installation suivant la revendication 4 ou 5, **caractérisée en ce que** ladite source lumineuse (31) est constituée d'une barrette comportant une pluralité de diodes électroluminescentes (Dₓ₁ - Dₓ₄) alignées parallèlement aux rangées d'alvéoles dans lesdits casiers (4a), et lesdits moyens de détection (30) comportant une pluralité de photo-détecteurs (CCD) dont la configuration spatiale est corrélée avec celle desdites diodes électroluminescentes (D11-D24),
lesdits moyens de pilotage sont programmés pour assurer automatiquement au moins deux cycles de mesure impliquant pour une même puissance lumineuse des durées d'illumination différentes, à savoir une durée relativement brève lors du premier cycle et une durée relativement longue lors du deuxième cycle.

7. Installation selon la revendication 6, **caractérisée en ce que** lesdites diodes électroluminescentes (Dₓ₁ - Dₓ₄) émettent dans l'infrarouge.

8. Installation selon la revendication 6 ou 7, **caractérisée en ce que** lesdites alvéoles (4X1 - 4X3) sont disposées selon une configuration en quinconce, **en ce que** le nombre desdites diodes électroluminescentes (Dx1 - Dx4) est le double de celui des alvéoles (4X1 - 4X3) desdites rangées (Rx) et **en ce qu'**elles sont commandées pour émettre en alternance lors du passage d'une rangée d'ordre pair à une rangée d'ordre impair.

9. Installation selon l'une quelconque des revendications 5 à 8, **caractérisée en ce que**, lesdits oeufs (OX1) étant susceptibles de présenter au moins un état supplémentaire auxdits états fécondé et clair, chaque état supplémentaire **se caractérisant par** une atténuation de la lumière traversant lesdits oeufs proche de celle associée au dit état fécondé, lesdits cycles d'émission de lumière comprennent un troisième cycle, de durée distincte desdits premier et deuxième cycles, ledit deuxième cycle discriminant les oeufs clairs des oeufs fécondés ou présentant un état supplémentaire, et **en ce que** ce troisième cycle comprend l'émission de lumière illuminant les alvéoles dont les coordonnées indiquent la présence d'un de ces états, d'une troisième durée, plus longue que ladite première durée, de manière à discriminer lesdits oeufs fécondés des dits oeufs présentant un troisième état, par détection d'atténuations différentes de la lumière traversant lesdits oeufs selon leur état, et la mise en mémoire de coordonnées des oeufs fécondés et/ou de troisième état, la dose d'illumination appliquée à chaque cycle étant choisie telle que l'on évite le risque d'éblouissement des moyens détecteurs sensibles aux faisceaux émergents.

10. Installation selon l'une quelconque des revendications 6 à 9, **caractérisée en ce qu'**elle comporte un dispositif de marquage desdits oeufs (5), par impression de taches (521a, 521b) de formes et/ou de couleurs déterminées, disposés à une position prédéterminée dudit dispositif de convoyage (2), et **en ce que** lesdits moyens d'analyse automatique (6) comprennent des moyens pour générer des signaux de commande sélective (51a, 61b) de ce dispositif de marquage (5), en relation temporelle avec l'avance desdits casiers (4a) sur ledit dispositif de convoyage (2) et en relation spatiale avec les coordonnées desdits oeufs (OX1) à marquer dans les casiers (4a) présentant au moins un desdits états à identifier.

## Patentansprüche

1. Ein Verfahren zur Analyse von Gegenständen, die an Orten gehalten werden, die eine sich wiederholende Verteilung von längsverlaufenden Linien und von querverlaufenden Reihen auf einem Förderer einhalten, der sie in Längsrichtung durch eine visiometrische Untersuchungsstation vorbeiziehend trägt, die Beleuchtungsmittel umfasst, welche bei der Untersuchung die genannten Orte in jeder Reihe durch einzelne einfallende Strahlen beleuchtet, und Sensormittel umfasst, die auf die austretenden Strahlen reagieren, die aus den Gegenständen weitergeleitet werden, wie auch Mittel umfasst zur Analyse durch Messung der Dämpfung für jeden Ort zwischen einfallendem Strahl und austretendem Strahl,
wobei die genannten Gegenstände Eier sind, die einzeln in Alveolen von Fächern (*casiers*) gehalten werden, die die genannten Orte bilden, wobei das Verfahren die Analyse jeder Reihe in mindestens zwei aufeinanderfolgenden Messzyklusschritten umfasst, indem die zu überprüfenden Alveolen durch unterschiedliche Beleuchtungsdosen bzw. -Mengen beleuchtet werden,
wobei man im ersten Schritt alle Alveolen der vorbeiziehenden Reihe beleuchtet, unter Bedingungen, die dazu geeignet sind, die Anwesenheit von leeren Alveolen zu erkennen und wobei man die Koordinaten ihres Standortes in der genannten vorbeiziehenden Reihe bestimmt und speichert, und
wobei man im zweiten Schritt nur die nicht leeren Alveolen der genannten Reihe beleuchtet, unter Bedingungen, die dazu geeignet sind, einen Zustand jedes einzelnen der genannten Eier durch Messung der Dämpfung zwischen einfallendem Strahl und austretendem Strahl für jede Alveole zu bestimmen.

2. Verfahren nach Anspruch 1, **dadurch gekennzeichnet, dass** die Beleuchtungsdosis, die bei jedem Schritt angewendet wird, durch Variation der Expositionsdauer bei einer bestimmten Lichtleistung gesteuert wird, wobei die Expositionsdauer für den ersten Schritt kürzer ist und für den zweiten Schritt länger ist.

3. Verfahren nach Anspruch 1 oder 2, wonach die jeweiligen Beleuchtungsbedingungen der zwei Schritte so gewählt werden, dass eine Blendung der Sensormittel vermieden wird, was die Analyse stören würde, indem man in zwei unterschiedlichen Empfindlichkeitsbereichen arbeitet, was entsprechend im genannten ersten Schritt erlaubt, von Eiern freie Alveolen von mit Eiern bestückten Alveolen zu unterscheiden, unabhängig davon, ob das jeweilige Ei befruchtet ist oder nicht, und was im genannten zweiten Schritt erlaubt, Alveolen, in denen das anwesende Ei befruchtet ist, von Alveolen zu unterscheiden, in denen das anwesende Ei nicht befruchtet ist.

4. Eine Einrichtung (1) zum Durchleuchten von Eiern, die Folgendes umfasst:
Fächer (4a) mit Alveolen, die die zu analysierenden Eier an Orten empfangen, die eine sich wiederholende Verteilung von längsverlaufenden Linien und von querverlaufenden Reihen einhalten,
ein Förderer (2) zum längs gerichteten Antrieb der genannten Fächer, um sie durch eine visiometrische Untersuchungsstation (5) laufen zu lassen,
wobei die visiometrische Untersuchungsstation (3) Folgendes umfasst:
eine Lichtquelle (31), die einen einfallenden Strahl erzeugt zur Beleuchtung von jedem einzelnen der genannten Eier jeder in der visiometrischen Untersuchungsstation vorbeiziehenden Reihe und
Sensormittel (30) zur Erfassung der austretenden Strahlen, die aus den genannten Eiern weitergeleitet werden,
wie auch Mittel zur automatischen Analyse durch Messung der erlittenen Dämpfung des Lichts zwischen einfallendem Strahl und austretendem Strahl für jeden Alveolen-Ort,
Steuerungsmittel (6) die die genannte Lichtquelle (31) und die genannten Sensormittel (30) synchron steuern, um jede Alveolen-Reihe mindestens zwei aufeinanderfolgenden Messzyklen unter unterschiedlichen Beleuchtungsbedingungen zu unterziehen, d. h.:
einem ersten Zyklus, in dem man alle Alveolen der vorbeiziehenden Reihe beleuchtet, indem man eine relativ niedrige Beleuchtungsdosis anwendet, in dem man abhängig von der Lichtdämpfung zwischen einfallendem Strahl und austretendem Strahl für jede Alveole bestimmt, ob es sich um eine leere Alveole, die also kein Ei enthält, handelt oder nicht, und wobei man die Koordinaten einer jeden leeren Alveole speichert, wodurch deren Anwesenheit in der genannten Reihe bestimmt wird,
und einem zweiten Zyklus, in dem man, durch das Anwenden einer relativ hohen Beleuchtungsdosis, nur die Alveolen der vorbeiziehenden Reihe beleuchtet, deren Koordinaten darauf hinweisen, dass ein Ei anwesend ist unter Bedingungen, die dazu geeignet sind, automatisch einen Zustand jedes einzelnen der genannten Eier abhängig von der erlittenen Dämpfung des Lichts zwischen einfallendem Strahl und austretendem Strahl für jede Alveole zu bestimmen.

5. Eine Einrichtung nach Anspruch 4, die konfiguriert ist, um die genannten Eier nach einem Zustand der Befruchtung und der Klarheit zu differenzieren, der sich anhand der von jedem Ei zwischen einfallendem Strahl und austretendem Strahl induzierten Dämpfung der Lichtleistung verdeutlicht, wie sie im Laufe des genannten zweiten Messzyklus bestimmt wird.

6. Eine Einrichtung nach Anspruch 4 oder 5, **dadurch gekennzeichnet, dass** die genannte Lichtquelle (31) aus einem LED-Stab mit einer Vielzahl an Leuchtdioden (Dₓ₁ - Dₓ₄) besteht, die parallel zu den Alveolen-Reihen in den genannten Fächern (4a) ausgerichtet sind, und dass die genannten Sensormittel (30) eine Vielzahl an Lichtsensoren (CCD) umfassen, deren räumliche Konfiguration mit der der genannten Leuchtdioden (D11 - D24) korreliert ist,
dass die genannten Steuerungsmittel programmiert sind, um automatisch mindestens zwei Messzyklen zu gewährleisten, was bei gleichbleibender Lichtleistung eine unterschiedliche Expositionsdauer impliziert, d. h. eine relativ kurze Dauer beim ersten Zyklus und einer relativ lange Dauer beim zweiten Zyklus.

7. Eine Einrichtung nach Anspruch 6, **dadurch gekennzeichnet, dass** die genannten Leuchtdioden (Dₓ₁ - Dₓ₄) im Infrarotbereich ausstrahlen.

8. Eine Einrichtung nach Anspruch 6 oder 7, **dadurch gekennzeichnet, dass** die genannten Alveolen (4X1 - 4X3) entsprechend einer gestaffelten Konfiguration angeordnet sind, dadurch, dass die Anzahl der genannten Leuchtdioden (Dₓ₁ - Dₓ₄) doppelt so groß ist wie die der Alveolen (4X1 - 4X3) der genannten Reihen (Rx) und dass diese auf eine Weise gesteuert werden, dass sie beim Übergang einer Reihe gerader Ordnung zu einer Reihe ungerader Ordnung alternierend ausstrahlen.

9. Eine Einrichtung nach irgendeinem der Ansprüche von 5 bis 8, **dadurch gekennzeichnet, dass** die genannten Eier (OX1), zusätzlich zum befruchteten Zustand und zum klaren Zustand, einen weiteren Zustand aufweisen können, wobei jeder zusätzliche Zustand durch eine Dämpfung des die genannten Eier durchlaufenden Lichts kennzeichnet ist, die in der Nähe derjenigen Dämpfung liegt, die mit dem genannten befruchteten Zustand verknüpft ist, wobei die genannten Zyklen der Lichtausstrahlung einen dritten Zyklus umfassen, dessen Dauer vom ersten und zweiten Zyklus verschieden ist, wobei der zweite Zyklus die klaren Eier von den befruchteten Eiern unterscheidet oder einen zusätzlichen Zustand aufweist, und dadurch, dass dieser dritte Zyklus eine Lichtausstrahlung umfasst, die die Alveolen beleuchtet, deren Koordinaten auf das Vorhandensein eines dieser Zustände schließen lassen, mit einer dritten Dauer, die länger als die genannte erste Dauer ist, wodurch die genannten befruchteten Eier von den Eiern, die den dritten Zustand aufweisen, unterschieden werden durch das Erfassen von unterschiedlicher Dämpfung von die Eier durchlaufendem Licht, gemäß deren Zustand, und das Speichern der Koordinaten der befruchteten und/oder im dritten Zustand befindliche Eier umfasst, wobei die Dosis der Beleuchtung, die bei jedem Zyklus angewendet wird, so gewählt wird, dass das Risiko einer Blendung der Sensormittel, die auf die austretenden Strahlen reagieren, vermieden wird.

10. Eine Einrichtung nach irgendeinem der Ansprüche von 6 bis 9, **dadurch gekennzeichnet, dass** sie eine Vorrichtung zur Markierung der genannten Eier (5) umfasst, durch Aufdrucken von Flecken (521a, 521b) mit bestimmten Formen und/oder Farben, die an einer vorbestimmten Position der genannten Fördervorrichtung (2) angeordnet sind, und dadurch, dass die gennannten Mittel zur automatischen Analyse (6) Mittel umfassen, um Signale zur selektiven Steuerung (51a, 61b) dieser Markierungsvorrichtung (5) zu erzeugen, im zeitlichen Zusammenhang mit dem Vorschub der genannten Fächer (4a) auf der genannten Fördervorrichtung (2) und im räumlichen Zusammenhang mit den Koordinaten der genannten Eier (OX1), die in den Fächern (4a) zu markieren sind, die mindestens einen der zu identifizierenden Zustände aufweist.

## Claims

1. Method for analysis of objects kept at locations having a repetitive distribution of longitudinal lines and transversal rows on a conveyor driving them to pass, in a longitudinal direction, through a visiometric examination station including lighting means lighting said locations in each row being examined by individual incidental beams and sensor means sensitive to the retransmitted emergent beams from said objects, as well as analysis means by measurement of attenuation between incidental beam and emergent beam for each location,
wherein said objects are eggs individually kept into rack cells constituting said locations, the method including the examination of each row in at least two steps of successive measurement cycles, by lighting cells to be examined with different lighting amounts,
wherein, in the first step, all the cells of the current row are lighted under conditions suitable to detect the presence of empty cells and the co-ordinates of their locations in said current row are determined and recorded in memory and,
in the second step, only the non-empty cells of said row are lighted under conditions suitable to determine a state of each one of said eggs by a measurement of attenuation between incidental beam and emergent beam for each cell.

2. Method according to claim 1, **characterized in that** the lighting amount applied at each step is adjusted by changing the exposure time under a given luminous power, the exposure time being shorter for first step and longer for second step.

3. Method according to claim 1 or 2 wherein the respective lighting conditions of the two steps are selected to avoid a dazzle of the sensor means that would disturb the analysis by operating within two different sensitivity ranges, respectively allowing, into said first step to distinguish the free eggs cells from those into which an egg is present, that it was or not fertilized, and into said second step to distinguish the cells into which the present egg is fertilized and those into which the present egg is not fertilized.

4. Installation (1) for eggs candling, including
racks (4a) with cells for receiving the eggs to be analyzed into locations laid out according to a repetitive configuration of longitudinal lines and transversal rows,
a conveyor (2) for driving in longitudinal direction said racks, to make them pass through a visiometric examination station (5),
the visiometric examination station (3) including :
a light source (31) generating an incidental beam for lighting each one of said eggs of each row successively passing within the visiometric examination station and
detection sensor means (30) for the emergent beams retransmitted by said eggs,
as well as automatic analysis means by measurement of the light attenuation undergone between incidental beam and emergent beam at each cell location,
control means (6) for synchronously control said light source (31) and said detection sensor means (30) to subject each cell row to at least two successive measurement cycles under different lighting amounts, namely :
a first cycle during which all the cells of the current row are lighted by applying a relatively low lighting amount, it is determined for each cell according to the attenuation of the light between incidental beam and emergent beam if it is or not an empty cell not containing any egg, and the co-ordinates of any empty cell whose presence is thus detected in said row are recorded into memory,
and a second cycle during which, by applying a relatively high lighting amount, the only cells of the current row whose co-ordinates indicate that an egg is present therein are lighted under conditions suitable to automatically determine a state of each one of said eggs according to the attenuation undergone by the light between incidental beam and emergent beam for each cell.

5. Installation according to claim 4, configured to discriminate said eggs following the fertilized and clear states according to the light intensity attenuation induced by each egg between incidental beam and emergent beam such as determined during said second measurement cycle.

6. Installation according to claim 4 or 5, **characterized in that** said light source (31) is made of a slide including a plurality of electroluminescent diodes (Dx1 - Dx4) lined in parallel to the cell rows in said racks (4a), and said detection sensor means (30) including a plurality of photo-detectors (CCD) whose spatial configuration is correlated with that of said electroluminescent diodes (D11-D24),
said controlling means are programmed to automatically ensure at least two measurement cycles implying for the same luminous power different lighting durations, namely a relatively short duration for the first cycle and a relatively long duration for the second cycle.

7. Installation according to claim 6, **characterized in that** said electroluminescent diodes (Dx1 - Dx4) emit an infrared light.

8. Installation according to claim 6 or 7, **characterized in that** said cells (4X1 - 4X3) are laid out according to an alternate rows configuration, **in that** the number of said electroluminescent diodes (Dx1 - Dx4) is twice as that of cells (4X1 - 4X3) of said rows (Rx) and **in that** they are controlled to emit alternately at the transition from an even row to an odd row.

9. Installation according to any of claims 5 to 8, **characterized in that**, said eggs (OX1) being likely to present at least an additional state from said fertilized and clear states, each additional state being identified by an attenuation of the light crossing said eggs close to that associated to said fertilized state, said light emitting cycles comprise a third cycle, of a duration distinct from that of said first and second cycles, said second cycle discriminating clear eggs from fertilized or presenting an additional state eggs, and **in that** this third cycle comprises the emission of light illuminating the cells whose co-ordinates indicate the presence of one of these states, of a third duration, longer than said first duration, so as to discriminate said fertilized eggs from said eggs being in a third state, by detection of different attenuations of the light crossing said eggs depending on their state, and the saving the co-ordinates of fertilized and/or in third state eggs in memory, the lighting amount applied at each cycle being selected such as to avoids the risk of dazzle of the detector means sensitive to the emergent beams.

10. Installation according to any of claims 6 to 9, **characterized in that** it includes a device for marking said eggs (5), by printing of spots (521a, 521b) of determined forms and/or colors, laid out at a predetermined position of said convoying device (2), and **in that** said automatic analysis means (6) comprise means intended to generate selective control signals (51a, 61b) for this marking device (5), in temporal relationship with the forwards movement of said racks (4a) on said convoying device (2) and in spatial relationship with the co-ordinates of said eggs (OX1) to be marked within the racks (4a) having at least one of said states to be identified.
